# EUROPEAN PATENT SPECIFICATION

(11) **EP 2 294 236 B1**
(45) Date of publication and mention of the grant of the patent: **08.04.2015**
(21) Application number: 09758679.6
(22) Date of filing: 21.05.2009
(51) Int. Cl.: C22C 23/04

(54) **RESORBABLE MAGNESIUM ALLOY**
RESORBIERBARE MAGNESIUMLEGIERUNG
ALLIAGE DE MAGNÉSIUM RÉSORBABLE

(30) Priority: 06.06.2008 US 59370
(43) Date of publication of application: 16.03.2011
(73) Proprietor: Synthes GmbH, 4436 Oberdorf (CH)
(72) Inventor: UGGOWITZER, Peter, CH-8913 Ottenbach (CH); GUNDE, Petra, CH-8606 Greifensee (CH); LOEFFLER, Joerg, CH-5425 Schneisingen (CH); IMWINKELRIED, Thomas, CH-4411 Seltisberg (CH); BECK, Stefan, CH-4133 Pratteln (CH); MONTALI, Andrea, CH-4055 Basel (CH)
(74) Representative: Carpmaels & Ransford LLP
(86) International application number: PCT/US2009/003138
(87) International publication number: WO 2009/148515

(56) References cited:
- WO-A1-2007/058276
- WO-A1-2008/035948
- US-A1- 2005 095 166
- US-A1- 2008 103 594

## Description

The invention relates to a magnesium alloy according to claim 1 and an implant made from the alloy according to claim 4.

In modem medical technology, implants are used for a broad range of applications, for example for orthopedic purposes, as support for vessels, and for attaching or fixing tissues or bones. Often the implants have only a temporary function until completion of the healing process. In order to avoid complications resulting from these implants remaining permanently in the body, they often must be operatively removed or made of a biocorrodible material which will be gradually degraded by the body. A growing number of such biocorrodible materials based on polymers or alloys are known. Of special interest are alloys made of biodegradable metals such as magnesium, iron and tungsten.

European Patent 1 270 023 describes a magnesium alloy which is reportedly suitable for the manufacture of endovascular or orthopedic implants. The alloy contains more than 50% magnesium and up to 5% rare earth metals. Other elements like aluminum, lithium and iron may also be contained in the disclosed alloy.

Application WO2008/035948 describes a biodegradable magnesium based alloy, comprising up to 40 atomic percent calcium as well as up to 40 atomic percent of one or more trace elements. The disclosed trace elements include Zr, Mo, Nb, Ta, Ti, Sr, Cr, Mn, Zn, Si, P, Ni and Fe. Through the addition of the trace elements the degradation rate of the magnesium alloy is reportedly varied.

Biodegradable magnesium alloys are also known to contain yttrium. WO02/100452 describes an alloy optionally comprising 0.01 to 7% by weight of yttrium and 0.01 to 8% by weight of rare earth metals. The alloy may also contain lithium and/or aluminum.

Ytterbium has been used as radiopaque marker element in implants. US2008/0033530 describes a marker alloy comprising 40 to 90 atomic percent of ytterbium, as well as 10 to 60 atomic percent magnesium and 0 to 10 atomic percent of one or several elements selected from the group of Ag, Zn, Au, Ga, Pd, Pt, Al, Sn, Ca, Nd, Ba, Si, and Ge. The alloy thus has a sufficient X-ray density at low material thicknesses. The degradation of ytterbium is reported as being approximately equal to the degradation of the main body.

Patent Application JP2004099940 describes a lightweight magnesium based alloy reportedly combining high strength with high ductility. The composition of the alloy comprises 0.5 to 5 atomic percent rare earth elements selected from Y, La, Ce, Pr, Nd, Pm, Sm, Eu, Gd, Tb, Dy, Ho, Er, Tm, Yb, Lu and miscl. metal. The alloy further comprises 0.2 to 4 atomic percent zinc.

A heat resistant magnesium alloy with 6-12% aluminum is known from US 2005/0095166.

The alloy according to the invention is related to a biocorrodible and age-hardenable alloy, comprised essentially of magnesium, ytterbium, zinc and calcium, and with optional components as defined in claim 1.

Inventive subject matter also includes an implant incorporating a magnesium alloy according to any one of claims 1 to 3.

According to the invention the magnesium alloy includes ytterbium, calcium and zinc. The three elements are present in following amounts:

| | |
|---|---|
| Ytterbium: | 0.5 to 8.0 weight percent |
| Zinc: | 0.2 to 6.0 weight percent |
| Calcium: | 0.1 to 2.0 weight percent |

The balance up to 100 weight percent includes magnesium as well as unavoidable impurities. For example, such impurities may stem from the production process of the alloy or from impurities already contained in the source material.

Surprisingly it was found that ytterbium containing magnesium alloys show a significantly increased age-hardenability if zinc and calcium are present. Moreover, such alloy embodiments also exhibit favorable corrosion properties in chlorine containing aqueous environments.

This alloy is produced, in some embodiments, by a micro-alloying process embodiment, such as is conventionally known. The micro-alloying process in combination with the chosen elements of embodiments described herein, which have a high grain growth restriction factor, enables the manufacture of an alloy having very good cold-forming properties and a low mechanical anisotropy. Composition described herein induce a fine grained structure in the alloy during solidification as well as during subsequent hot-forming processes. This is mainly due to the formation of fine precipitations of these elements which restrict undesirable grain growth during recrystallization.

According to the invention the magnesium alloy as described herein may optionally contain further elements, namely manganese, zirconium, aluminum, scandium and yttrium in the amounts indicated in claim 1.

An implant containing the alloy as disclosed herein may be produced using techniques known in the art. The implant may be in any form, especially in the form of a plate, specifically a bone plate, a screw, a nail, a bone nail, a stent, a rod. Implants made of the specified alloy are suitable for implantation in animal or human body.

### EXAMPLE

Novel magnesium alloys containing 4 wt-% Yb, 0.8 wt-% Zn and 0.25 wt% Ca were melted and cast in an induction furnace in Ar-atmosphere. The billets were extruded at a temperature of 350°C to an end-diameter of 8.6 mm, which corresponds to an extrusion-ratio of 12.5. The microstructure of the extruded alloys showed a very fine-grained structure with a grain size of approximately 5 µm. This material featured an average yield strength of 150 MPa, tensile strength of 250 MPa, uniform elongation of 20% and elongation to fracture of 28%. Hardness measurements indicated an age hardening response, where the values increased from approximately 50 HV5 in the solution heat-treated state to approximately 70 HV5 in the age hardened state.

## Claims

1. A magnesium alloy, consisting of, by percentage of weight:
0.5 to 8.0 percent ytterbium;
0.1 to 2.0 percent calcium; and
0.2 to 6.0 percent zinc;
and optionally
up to 4.0 percent scandium;
up to 2.0 percent yttrium;
0.05 to 1.0 percent manganese;
up to 1.0 percent zirconium; and
up to 2.0 percent aluminum,
wherein the balance up to 100% by weight is magnesium.

2. The alloy of claim 1, wherein the alloy has a grain structure with grains of approximately 5 µm.

3. The alloy of any one of claims 1 or 2, wherein the alloy has an age hardened hardness value of approximately 70 on a Vickers HV5 scale.

4. An implant comprising a magnesium alloy according to any one of claims 1 to 3.

## Patentansprüche

1. Magnesiumlegierung, bestehend aus:
0,5 bis 8,0 Gewichtsprozent Ytterbium;
0,1 bis 2,0 Gewichtsprozent Calcium und
0,2 bis 6,0 Gewichtsprozent Zink
und gegebenenfalls
bis zu 4,0 Gewichtsprozent Scandium;
bis zu 2,0 Gewichtsprozent Yttrium;
0,05 bis 1,0 Gewichtsprozent Mangan;
bis zu 1,0 Gewichtsprozent Zirconium und
bis zu 2,0 Gewichtsprozent Aluminium,
wobei es sich bei dem Rest auf 100 Gew.-% um Magnesium handelt.

2. Legierung nach Anspruch 1, wobei die Legierung eine Kornstruktur mit Körnern mit einer Größe von ungefähr 5 µm aufweist.

3. Legierung nach einem der Ansprüche 1 oder 2, wobei die Legierung einen Härtewert nach Ausscheidungshärtung von ungefähr 70 auf einer Vickers-HV5-Skala aufweist.

4. Implantat, umfassend eine Magnesiumlegierung nach einem der Ansprüche 1 bis 3.

## Revendications

1. Alliage de magnésium, constitué, en pourcentage en poids :
de 0,5 à 8,0 % d'ytterbium ;
de 0,1 à 2,0 % de calcium ; et
de 0,2 à 6,0 % de zinc ;
et éventuellement
de jusqu'à 4,0 % de scandium ;
de jusqu'à 2,0 % d'yttrium ;
de 0,05 à 1,0 % de manganèse ;
de jusqu'à 1,0 % de zirconium ; et
de jusqu'à 2,0 % d'aluminium,
le reste jusqu'à 100 % en poids étant constitué par du magnésium.

2. Alliage selon la revendication 1, dans lequel l'alliage a une structure granulométrique avec des grains d'environ 5 µm.

3. Alliage selon l'une quelconque des revendications 1 ou 2, dans lequel l'alliage a une valeur de dureté durcie par vieillissement d'environ 70 sur une échelle Vickers HV5.

4. Implant comprenant un alliage de magnésium selon l'une quelconque des revendications 1 à 3.
